# EUROPEAN PATENT SPECIFICATION

(11) **EP 3 294 358 B1**
(45) Date of publication and mention of the grant of the patent: **15.04.2020**
(21) Application number: 16725324.4
(22) Date of filing: 13.05.2016
(51) Int. Cl.: A61L 29/08, A61L 29/16, A61L 31/10, A61L 31/16

(54) **DRUG COATED MEDICAL DEVICES**
WIRKSTOFFBESCHICHTETE MEDIZINISCHE VORRICHTUNGEN
DISPOSITIFS MÉDICAUX REVÊTUS DE MÉDICAMENT

(30) Priority: 13.05.2015 US 201562161166 P
(43) Date of publication of application: 21.03.2018
(73) Proprietor: Boston Scientific Scimed Inc., Maple Grove, Minnesota 55311 (US)
(72) Inventor: KANGAS, Steven L., Woodbury, Minnesota 55125 (US)
(74) Representative: Peterreins Schley
(86) International application number: PCT/US2016/032319
(87) International publication number: WO 2016/183421

(56) References cited:
- US-A1- 2010 312 182
- US-A1- 2014 221 522
- US-A1- 2014 271 774
- US-A1- 2014 277 399
- WENDA C. CARLYLE ET AL: "Enhanced drug delivery capabilities from stents coated with absorbable polymer and crystalline drug", JOURNAL OF CONTROLLED RELEASE., vol. 162, no. 3, 1 September 2012 (2012-09-01), pages 561-567, XP055289527, NL ISSN: 0168-3659, DOI: 10.1016/j.jconrel.2012.07.004

## Description

### Field

The present disclosure pertains to medical devices, and methods for manufacturing medical devices. More particularly, the present disclosure pertains to medical devices for therapeutic agent delivery.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

### Brief Summary

This disclosure provides design, material, manufacturing method, and use alternatives for medical devices. The expandable medical device comprises:
a therapeutic coating comprising a polydopamine coated therapeutic agent disposed on a surface of the expandable medical device.

Additionally to the embodiment above, the therapeutic agent may be selected from the group consisting of paclitaxel, everolimus, rapamycin, sirolimus, tacrolimus, heparin, diclofenac, aspirin, and any combination thereof.

Alternatively or additionally to the previous embodiment, the therapeutic agent may have an average particle size in the range of 0.1 - 20 micrometers.

Alternatively or additionally to any of the embodiments above, the polydopamine may have a thickness in the range of 1- 40 nanometers.

Alternatively or additionally to any of the embodiments above, the expandable medical device may be a balloon catheter.

The balloon catheter may comprise:
a catheter shaft comprising an inner tubular member and an outer tubular member, the catheter shaft extending from a proximal end to a distal end; and
an inflatable balloon having a proximal end affixed to the outer tubular member of the catheter shaft and a distal end affixed to the inner tubular member of the catheter shaft proximal to the distal end of the catheter shaft, the inflatable balloon disposed adjacent to the distal end of the catheter shaft.

Additionally, the therapeutic coating may be disposed over an entire outer surface area of the inflatable balloon or alternatively, the therapeutic coating may be disposed over less than an entire outer surface area of the inflatable balloon.

The expandable medical device can also be a stent.

The stent may comprise:
an elongated tubular member having a first end, a second end and an intermediate region disposed therebetween, the elongated tubular member comprising at least one filament.

Additionally, the therapeutic coating may be disposed on an outer surface of the stent.

Additionally, the therapeutic coating may be disposed on an inner surface of the stent.

Additionally, the therapeutic coating may be disposed over an entire surface area of the stent or alternatively, the therapeutic coating may be disposed over less than an entire surface area of the stent.

Alternatively or additionally to any of the embodiments above, the therapeutic coating binds with primary amines.

The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1A is cross-sectional view of an illustrative medical device;
FIG. 1B is a side view of an illustrative endoluminal implant;
FIG. 2 is the chemical structure of dopamine;
FIG. 3 is a schematic of an illustrative polymerization reaction;
FIG. 4 is a photograph of dispersions of varying polymerization times of polydopamine coated everolimus;
FIG. 5 is a graph of the everolimus content of everolimus-polydopamine particles as a function of polymerization time;
FIG. 6A is a microscope image of an everolimus dispersion;
FIG. 6B is a microscope image of an everolimus-polydopamine dispersion;
FIG. 7A is a scanning electron microscope image of everolimus particles;
FIG. 7B is a scanning electron microscope image of everolimus- polydopamine particles;
FIG. 8 is a graph of the dissolution profile of everolimus and everolimus-polydopamine;
FIG. 9 is a photograph of various coatings removed from drug coated balloons;
FIG. 10 is another photograph of various coatings removed from drug coated balloons;
FIG. 11 is a schematic view of an illustrative polydopamine coated microparticle bonded to a vessel wall;
FIG 12A is a microscope image of a control microsphere before a rinse;
FIG. 12B is a microscope image of a control microsphere after a rinse;
FIG. 12C is a microscope image of a polydopamine coated microsphere before a rinse; and
FIG. 12D is a microscope image of a polydopamine coated microsphere after a rinse.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the disclosure.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

The body includes various passageways such as blood vessels and body lumens. These passageways sometimes become occluded (for example, by a tumor or plaque). To widen an occluded body vessel, balloon catheters can be used, for example, in angioplasty.

In some embodiments, a balloon catheter can include an inflatable and deflatable balloon carried by a long and narrow catheter body. The balloon can be initially folded around the catheter body to reduce the radial profile of the balloon catheter for easy insertion into the body. During use, the folded balloon can be delivered to a target location in the vessel, for example, a portion occluded by plaque, by threading the balloon catheter over a guide wire previously located in the vessel. The balloon is then inflated, for example, by introducing a fluid (such as a gas or a liquid) into the interior of the balloon. Inflating the balloon can radially expand the vessel so that the vessel can permit an increased rate of blood flow. After use, the balloon is typically deflated and withdrawn from the body. In some instances, it may be desirable to coat, layer, or otherwise apply a drug or therapeutic agent to an outer surface of the balloon to deliver and/or administer the drug or therapeutic agent to a lumen wall when the balloon is expanded. However, it may be difficult to deposit certain therapeutic agents on the surface of certain balloons. While the therapeutic coating described herein is discussed relative to balloons and balloon catheters, it is contemplated that the therapeutic coating can be applied to and/or used in conjunction with other medical devices, such as, but not limited to, stents, embolic filters, implantable devices, treatment devices, etc.

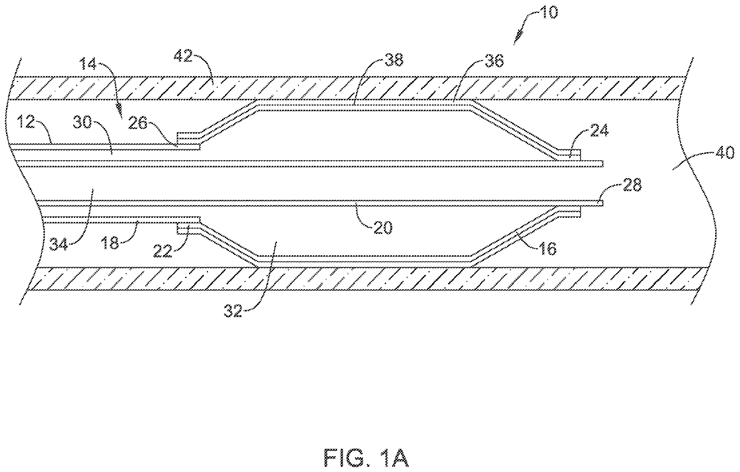

Figure 1A is a cross-sectional side view of a distal end region of an example medical device 10 that, in this example, takes the form of a catheter, disposed in a body lumen 40. In at least some embodiments, the catheter 10 may be a balloon catheter. The catheter 10 may include an elongate catheter shaft 12 having a proximal end (not shown) and a distal end region 14. The catheter shaft 12 may extend proximally from the distal end region 14 to the proximal end which is configured to remain outside of a patient's body. Although not shown, the proximal end of the catheter shaft 12 may include a hub attached thereto for connecting other treatment devices or providing a port for facilitating other treatments. It is contemplated that the stiffness and size of the catheter shaft 12 may be modified to form a catheter 10 for use in various locations within the body. The catheter 10 may be configured to be advanced through a guide sheath, delivery sheath, or other guide means.

The catheter 10 may further include an inflatable balloon 16 affixed adjacent to the distal end region 14 of the catheter shaft 12. The size of the balloon 16 may vary based on where in the body it is used (e.g., along the coronary and/or peripheral vasculature, in a pulmonary vessel, along an airway, along another body lumen, or the like). The balloon 16 may have an outer diameter (in the inflated state) in the range of 1 millimeter (mm) to 26 mm, or about 2 to 10 mm, or about 2.5 to 8 mm. The balloon 16 may have a length in the range of 5 mm to 300 mm, or about 5 to 100 mm, or about 10-50 mm. The balloon 16 may have a wall thickness in the range of 10 micrometers (µm) to 100 µm, or about 10 to 75 µm, or about 10 to 50 µm. The catheter shaft 12 may include an outer tubular member 18 and an inner tubular member 20. A proximal waist 22 of the balloon 16 may be secured to a distal end region 26 of the outer tubular member 18. A distal waist 24 of the balloon 16 may be secured to a distal end region 28 of the inner tubular member 20. The inner tubular member 20 may extend distally beyond the distal waist 24 of the balloon 16, although this is not required. In some instances, an annular inflation lumen 30 may be disposed between the outer tubular member 18 and the inner tubular member 20. The inflation lumen 30 may allow inflation fluid to pass from an inflation fluid source configured to remain outside the body to the interior region 32 of the balloon 16. The inner tubular member 20 may further define a lumen 34 through which a guidewire (not explicitly shown) may be passed in order to advance the catheter 10 to a predetermined position, although this is not required.

In some embodiments, an outer surface 38 of the balloon 16 may be coated with or otherwise include an elutable drug or coating 36. The coating 36 can be substantially (e.g., about 60% or more, about 95% or more, about 98% or more, about 99% or more, about 100%) formed of one or more therapeutic agents. The coating can be substantially (e.g., about 60% or more, about 95% or more, about 98% or more, about 99% or more, about 100%) free of a polymer or low molecular weight organic matrix (e.g., a polymeric or low molecular weight matrix in which the therapeutic agent may be incorporated), although this is not required. As used herein, "about" or "approximately" can refer to a margin of error of ± 2% of a given numerical value or ratio. A coating without a polymer or low molecular weight matrix can decrease the likelihood of adverse bodily reactions to the polymer or low molecular weight matrix and/or or its degradation products. With the presence of a polymer or low molecular weight organic matrix, the drug coating can release a greater amount of drug in a shorter amount of time.

The terms "therapeutic agents," "drugs," "bioactive agents," "pharmaceuticals," "pharmaceutically active agents", and other related terms may be used interchangeably herein and include genetic therapeutic agents, non-genetic therapeutic agents, and cells. Therapeutic agents may be used singly or in combination. A wide range of therapeutic agent loadings can be used in conjunction with the devices of the present invention, with the pharmaceutically effective amount being readily determined by those of ordinary skill in the art and ultimately depending, for example, upon the condition to be treated, the nature of the therapeutic agent itself, the tissue into which the dosage form is introduced, and so forth.

Some specific beneficial agents include anti-thrombotic agents, anti-proliferative agents, anti-inflammatory agents, anti-migratory agents, agents affecting extracellular matrix production and organization, antineoplastic agents, anti-mitotic agents, anesthetic agents, anti-coagulants, vascular cell growth promoters, vascular cell growth inhibitors, cholesterol-lowering agents, vasodilating agents, and agents that interfere with endogenous vasoactive mechanisms.

More specific drugs or therapeutic agents include paclitaxel, rapamycin, sirolimus, everolimus, tacrolimus, heparin, diclofenac, aspirin, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, and Serca 2 gene/protein, resiquimod, imiquimod (as well as other imidazoquinoline immune response modifiers), human apolioproteins (e.g., AI, AII, AIII, AIV, AV, etc.), vascular endothelial growth factors (e.g., VEGF-2), as well as derivatives of the forgoing, among many others.

Numerous additional therapeutic agents useful for the practice of the present invention may be selected from those described in paragraphs [0040] to [0046] of commonly assigned U.S. Patent Application Pub. No. 2003/0236514.

In some instances, drug coated balloons (DCB) have involved coating everolimus on the balloon from an organic solvent. The resulting amorphous coating was vapor annealed in ethanol vapor to convert the amorphous everolimus to the less soluble crystalline form. A preclinical animal study showed significant tissue levels of everolimus at 28 days for DCB using the vapor annealing process (greater drug levels than observed with other eluting balloons). However, the vapor annealing process may have some drawbacks. For example, the vapor annealing process may damage some balloons. It may be desirable to deposit crystalline everolimus directly on the balloon, thereby eliminating the vapor annealing step. In some instances, microcrystalline everolimus particles may be dispersed in water. However, it may not be possible to coat this dispersion using the traditional commercial syringe coating processes. For example, the everolimus particles in the dispersion may flocculate (aggregate), resulting in settling of the dispersion in the lines of the coater and plugging of the lines. A number of surfactants were evaluated in an attempt to stabilize the dispersion, however none were effective at stabilizing the everolimus drug particles.

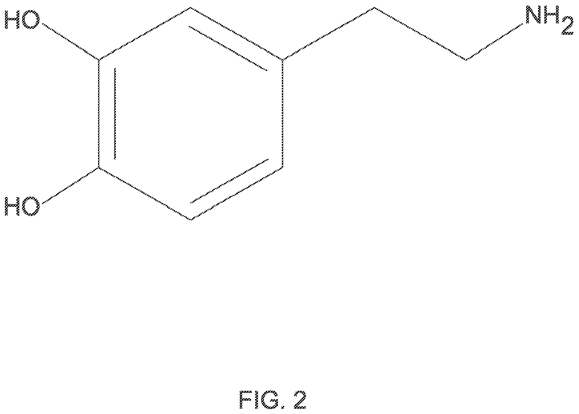

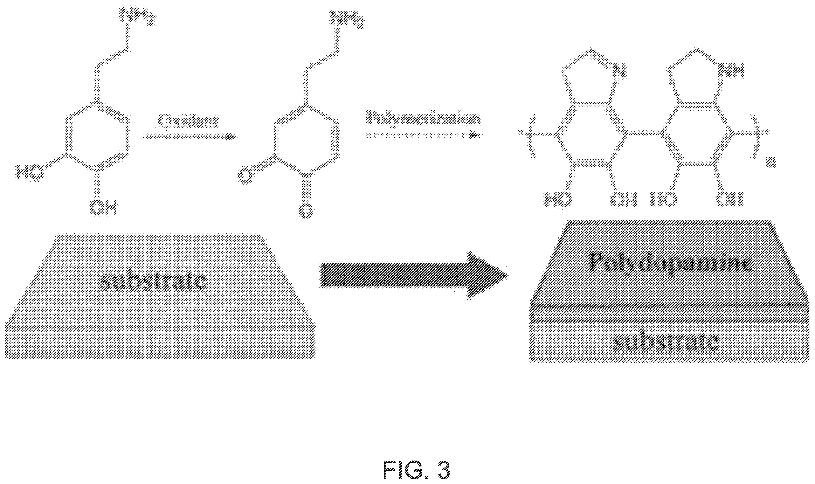

Relatively recent research on understanding the underlying mechanism of the excellent wet adhesion observed with Mussels on various substrates has shown that the adhesive protein secreted by Mussels is high in the catechol DOPA (3,4-dihydroxyphenyl-L-alanine) (Mussel-Inspired Adhesives and Coatings. Annual Review of Materials Research. 2011. 41:90-132). The catechol group is able to strongly interact with the substrate through very strong non-covalent and covalent bonding under wet conditions. Subsequent work focused on the preparation of synthetic biomimetic mussel like adhesives has involved the synthesis of polymers containing the catechols DOPA (or dopamine). Figure 2 shows the structure of dopamine. It has been observed that aqueous solutions of dopamine at a basic pH (in the range of 8-9 or approximately 8.5) will oxidize and spontaneous polymerize at room temperature (Mussel-Inspired Surface Chemistry for Multifunctional Coatings. SCIENCE 2007. 318:426-430). Polydopamine may deposit on any solid surface present in the solution during polymerization. The resulting coating thickness may be self-limiting at about 40 nanometers (nm) and the coating is tightly adhered to the substrate. This may be due to strong non-covalent and covalent bonding through the catechol groups. The resulting polydopamine coating can be functionalized with molecules containing primary amine groups. For example, polydopamine coatings may covalently bind with proteins containing primary amines under physiological conditions. While the structure of polydopamine is not precisely known, it is believed to be the same as, or similar to, eumelanin - the pigment contained in human skin. Figure 3 illustrates the spontaneous polymerization of dopamine to polydopamine. Research has shown polydopamine to be biocompatible (Polydopamine Coating Enhance Biointegration of a Model Polymeric Implant. Soft Matter 2011. 7:8305-8312).

It is contemplated that a microcrystalline drug particle, such as, but not limited to, everolimus, can be coated with polydopamine. The polydopamine coating may impart several properties to the everolimus particles. For example, the polydopamine coating may improve the bonding of the drug particle to a target tissue site. In addition, polydopamine coating may impart several unexpected properties to the everolimus particles. For example, the polydopamine coating may desirably reduce or eliminate foaming of the aqueous drug dispersion. The polydopamine coating may also desirably reduce or eliminate agglomeration of the everolimus microparticles in a dispersion. The observed ability of polydopamine to reduce foaming and agglomeration of the everolimus particles was unexpected. These properties may make it possible to coat the dispersion onto a balloon, such as balloon 16 shown in Figure 1A, or other medical device, without settling of the particles and/or clogging of the tubing used in the coating process. It is further contemplated that the polydopamine coating may also cause a reduction in large particulates after balloon inflation and impart the ability of the particles to covalently bind to the artery thereby increasing drug transfer efficiency.

In some instances, the elutable drug coating 36 may include polydopamine coated everolimus microparticles. However, it is contemplated that the drug coating 36 may comprise other polydopamine coated drugs, such as, but not limited to those disclosed herein. It is contemplated that the polydopamine may surround or encapsulate the everolimus microparticles. The everolimus microparticles may have an average particle size in the range of 0.1 - 20 micrometers (µm), 1 - 20 µm, or 5 - 15 µm. The polydopamine coating may have a thickness in the range of 1- 40 nanometers (nm), 10 - 30 nm, or 10 - 20 nm. It is contemplated that the thickness of the polydopamine coating may be determined and controlled by the incubation time and/or the concentration of dopamine solution (for example, the amount of time dopamine is allowed to polymerize onto a substrate). The elutable drug 36 may be coated onto the balloon 16 in any thickness desired to achieve the desired drug concentration. For example, the elutable drug coating 36 may have a thickness in the range of 1 - 20 µm, 5 - 15 µm, or 7 - 13 µm. These are just examples.

In some embodiments, the elutable drug coating 36 may cover the entire surface area 38 of the balloon 16. In other embodiments, the elutable drug coating 36 may cover a portion of the surface area of the balloon 16. For example, the elutable drug coating 36 may cover 90% or less (e.g., about 80% or less, about 70% or less, about 60% or less, about 50% or less, about 40% or less, about 30% or less, about 20% or less, or about 10% or less) and/or about 10% or more (e.g., about 20% or more, about 30% or more, about 40% or more, about 50% or more, about 60% or more, about 70% or more, 80% or more, or 90% or more) of the balloon's outer surface 38.

In some embodiments, the drug elutable coating 36 may optionally include a polymer or low molecular weight organic compound. The polymer or compound can be biodegradable. The polymer or organic compound can, for example, provide structural support for a therapeutic agent coating 36 that may be fragile, and/or can slow or speed up the release of a therapeutic agent within the coating. The coating 36 can include about 90% or less (e.g., about 75% or less, about 50% or less, about 25% or less, about 10% or less, about 5% or less or 1% or less) and/or about 5% or more (e.g., about 10% or more, about 25% or more, about 50% or more, or about 75% or more) by weight of a polymer or organic compound. The polymer or organic compound can form a homogeneous mixture with one or more therapeutic agents within the coating, or can be a separate layer over or under the one or more therapeutic agents. In some embodiments, the polymer or organic compound can form a porous network that intertwines with a layer of one or more therapeutic agents.

In some embodiments, the catheter 10 may be advanced through a lumen 40 having a lumen wall 42, with or without a guidewire, or other guide means. When the balloon 16 is positioned adjacent to the desired treatment region, the balloon 16 may be expanded to bring the elutable drug coating 36 into contact with the lumen wall 42. The drug coating 36 may elute a therapeutic agent, such as, but not limited to, polydopamine coated everolimus microparticles to the body lumen wall 42. The balloon 16 may remain inflated for a desired amount of time sufficient to transfer the coating 36 from the balloon 16 to the wall 42.

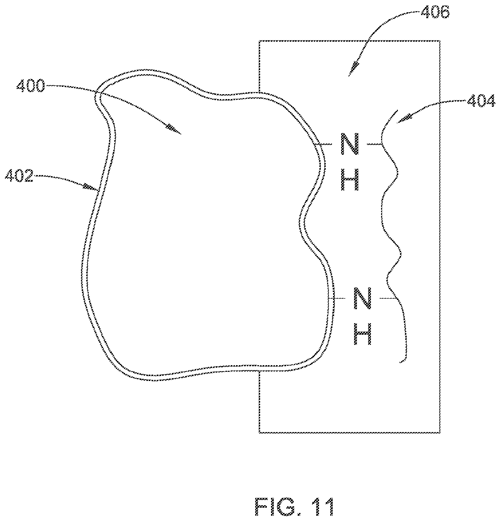

Polydopamine coatings can bind proteins that contain primary amine functional amino acids (Improving the Blood Compatibility of Material Surfaces Via Biomolecule-Immobilized Mussel-Inspired Coatings. Journal of Biomedical Materials Research A. 2011. Vol. 96. Issue 1: 38-45). Everolimus-polydopamine particles from a deployed drug coated balloon may also bind with proteins on the surface of the artery such as, for example, collagen (in the case of in-stent restenosis). This may result in anchoring of the particle to the vessel wall to reduce particle wash-out and hence increase transfer efficiency. This is shown schematically in Figure 11. The everolimus microparticle 400 may have a thin polydopamine coating 402. The polydopamine coating 402 may bind with the proteins 404 in the vessel wall 406.

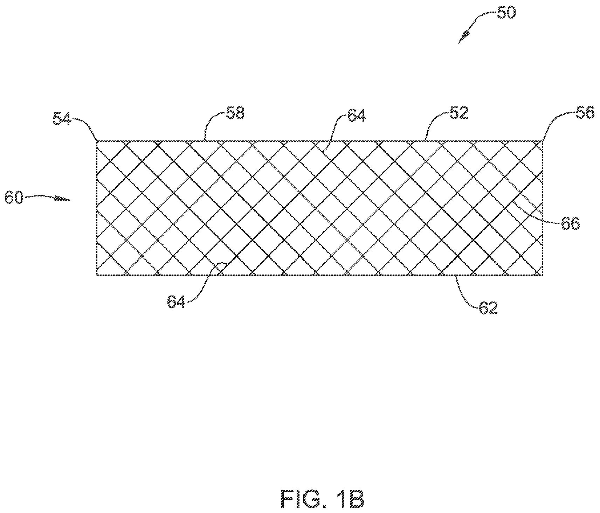

Figure 1B illustrates a side view of an illustrative endoluminal implant 50, such as, but not limited to, a stent. In some instances, the stent 50 may be formed from an elongated tubular member 52. While the stent 50 is described as generally tubular, it is contemplated that the stent 50 may take any cross-sectional shape desired. The stent 50 may have a first, or proximal, end 54 and a second, or distal, end 56. The stent 50 may include a lumen 60 extending from a first opening adjacent the first end 54 to a second opening adjacent to the second end 56 to allow for the passage of food, fluids, etc. In some embodiments, an outer surface 62 of the stent 50 may be coated with or otherwise include an elutable drug or coating 66. The coating 66 may be similar in form and function to the elutable drug coating 36 described herein. Alternatively, or additionally, the coating 66 may be coated onto an inner surface of the stent 50.

In some embodiments, the elutable drug coating 66 may cover the entire surface area of the stent 50. In other embodiments, the elutable drug coating 66 may cover a portion of the surface area of the stent 50. For example, the elutable drug coating 66 may cover 90% or less (e.g., about 80% or less, about 70% or less, about 60% or less, about 50% or less, about 40% or less, about 30% or less, about 20% or less, or about 10% or less) and/or about 10% or more (e.g., about 20% or more, about 30% or more, about 40% or more, about 50% or more, about 60% or more, about 70% or more, 80% or more, or 90% or more) of the stent 50.

The stent 50 may be expandable from a first collapsed configuration (not explicitly shown) to a second expanded configuration. The stent 50 may be structured to extend across a structure and to apply a radially outward pressure to the stricture in a lumen to open the lumen and allow for the passage of foods, fluids, air, etc.

The stent 50 may have a woven structure, fabricated from a number of filaments or struts 64. In some embodiments, the stent 50 may be braided with one filament. In other embodiments, the stent 50 may be braided with several filaments, as is found, for example, in the WallFlex®, WALLSTENT®, and Polyflex® stents, made and distributed by Boston Scientific, Corporation. In another embodiment, the stent 50 may be knitted, such as the Ultraflex™ stents made by Boston Scientific, Corporation. In yet another embodiment, the stent 50 may be of a knotted type, such the Precision Colonic™ stents made by Boston Scientific, Corporation. In still another embodiment, the stent 50 may be a laser cut tubular member, such as the EPIC™ stents made by Boston Scientific, Corporation. A laser cut tubular member may have an open and/or closed cell geometry including one or more interconnected filaments. In some instances, an inner and/or outer surface of the stent 50 may be entirely, substantially or partially, covered with a polymeric covering or coating. For example, a covering or coating which may help reduce food impaction and/or tumor or tissue ingrowth.

### Example 1

The disclosure may be further clarified by reference to the following Examples, which serve to exemplify some of the embodiments, and not to limit the disclosure.

To prepare a microcrystalline everolimus dispersion, amorphous everolimus was dissolved in isopropyl alcohol at 40 weight percent with gentle warming at 40 degrees Celsius (°C). The solution was allowed to sit at room temperature overnight resulting in crystallization of the everolimus. The large crystals were dried under vacuum at room temperature. 0.1 grams (g) of crystalline everolimus, 0.16 g of water and 1.85 g of 100 micrometer (µm) zirconia (Zr) beads were added to a stainless steel ampule. The ampule was sealed and placed on a high speed amalgamator shaker for 20 min. Water (2 milliliters (mL)) was added to the resulting paste and the mixture was swirled to disperse the milled everolimus particles.

The water/everolimus dispersion was decanted off from the Zr beads and filtered through a 30 µm nylon mesh filter. The Zr bead slurry was then washed an additional three times using about 2 mL water each time and each time the water/everolimus dispersion was filtered through 30 µm nylon mesh filter. The combined (from each wash) filtered dispersion was then centrifuged at 4,000 rpm for 10 minutes. The supernatant was decanted off until there was about 1-2 mL of liquid remaining in the centrifuge tube along with the everolimus particles resulting in a microcrystalline everolimus dispersion in water.

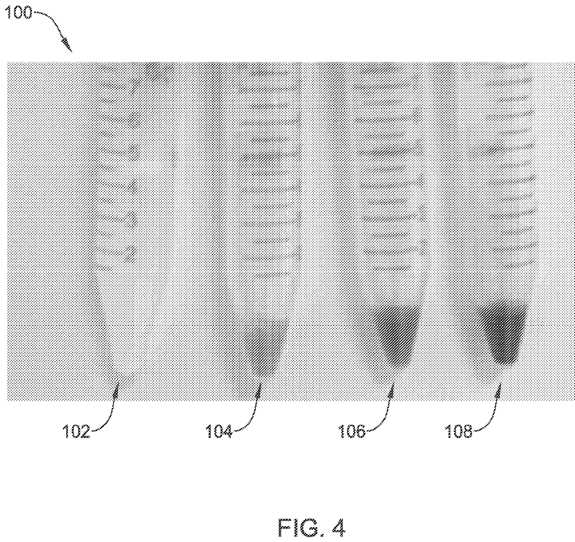

To prepare the polydopamine coated everolimus particles, the microcrystalline everolimus dispersion in water from above was washed/centrifuged two to three times with a solution of dopamine dissolved in tris(hydroxymethyl) aminomethane (THAM) buffer at pH 8.5 with a concentration of 2 milligrams (mg) of dopamine per milliliter of buffer. After the final wash, about 10 mL of the dopamine solution was left in the centrifuge tube and the tube placed on a shaker at 150 rpm for 1 hour at room temperature. Additional 10 mL samples of the dopamine solution were left in the centrifuge tube and the tube placed on a shaker at 150 rpm for 2, 4, and 24 hours at room temperature. The incubation times allow the dopamine to polymerize on the everolimus particles, forming a polydopamine coated everolimus particle. The resulting dispersions were washed two to four times with deionized water and centrifuged to remove the excess dopamine solution. The resulting dispersion was 2-5% (weight) solids. The dispersion is bronze in color due to the polydopamine coating. Figure 4 is a photograph 100 of the dispersions of varying polymerization (incubation) times of polydopamine coated everolimus. A control everolimus dispersion 102 is shown on the far left. As can be seen, the dispersion is substantially colorless. An everolimus/polydopamine dispersion that was placed on the shaker for one hour is shown at 104. An everolimus/polydopamine dispersion that was placed on the shaker for four hours is shown at 106. An everolimus/polydopamine dispersion that was placed on the shaker for 24 hours is shown at 108. The polydopamine coating thickness increases with incubation time which can be observed with the increase in color of the dispersion with time, as shown in Figure 4.

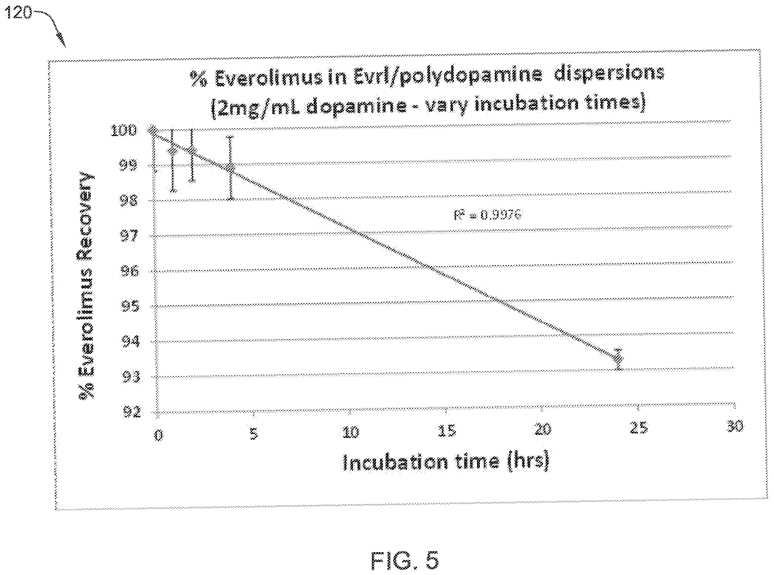

The amount of polydopamine in the dispersion (everolimus/polydopamine ratio) was determined by high performance liquid chromatography (HPLC). The dispersion was dried to remove the water (forming everolimus/polydopamine particles). The particles were dissolved in tetrahydofuran (THF) and injected into the HPLC. The results are shown in Figure 5, which is a graph 120 of the percent of everolimus weight/weight (% wt/wt) in the everolimus/polydopamine particles as a function of incubation times. As can be seen the graph 120, for everolimus incubated in a dopamine solution for less than 4 hours the dopamine content was approximately less than 1% (wt/wt). At a 24 hour incubation the polydopamine content was about 7%.

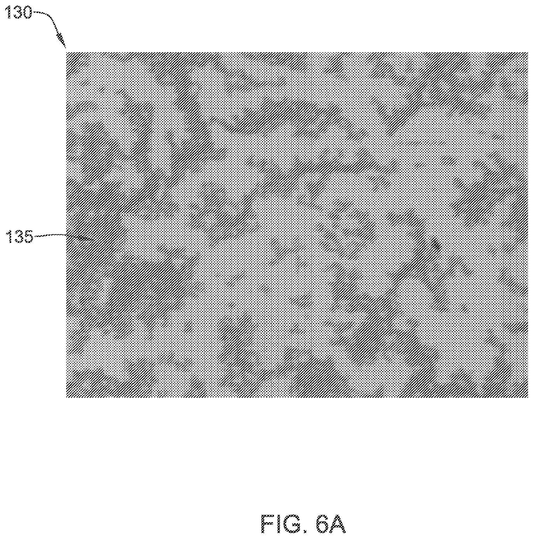

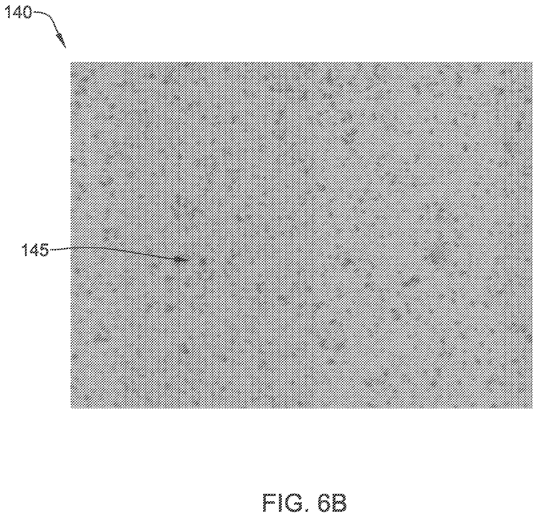

Figure 6A is an optical micrograph 130 of an everolimus dispersion. As can be seen, the everolimus control particles 135, which appear darker than the background, are agglomerated into large clusters. Figure 6B is an optical micrograph 140 of an everolimus/polydopamine dispersion that was prepared according to the procedure above and allowed to incubate for one hour. As can be seen, the everolimus/polydopamine particles 145, which appear darker than the background, shows no agglomeration of the particles 145. Thus, polydopamine is effective at stabilizing the everolimus microparticles from agglomeration. Attempts to coat medical device balloons, such as the balloon 16 described above, using a commercial syringe coater was not possible with the everolimus control dispersion. Agglomeration of the dispersion in the line resulted in settling/separation of the drug particles and clogging of the line at tube connector junctions. However, coating of balloons using the commercial syringe coater was possible using the everolimus-polydopamine dispersion due to stabilization of the dispersion by the polydopamine coating.

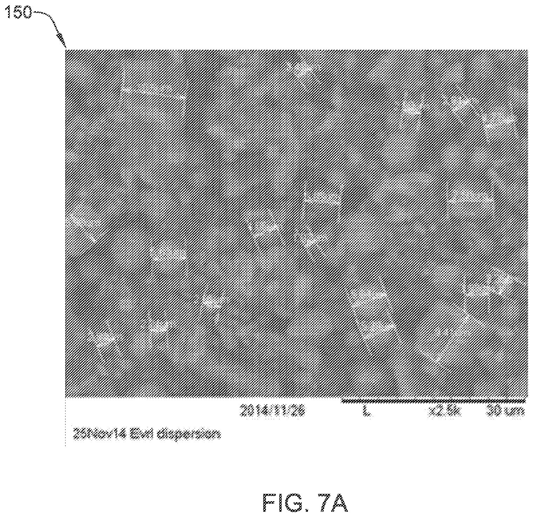

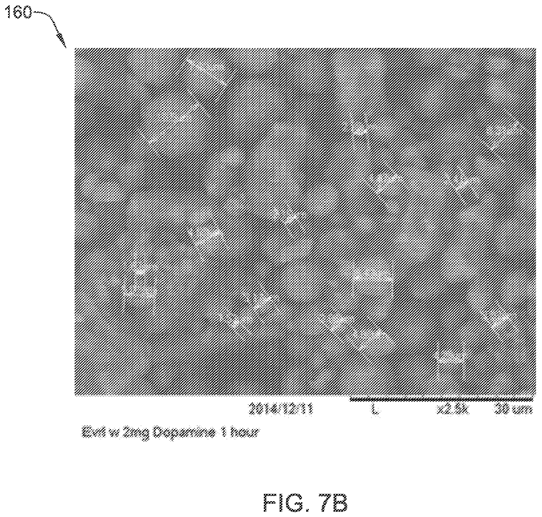

Figure 7A is a scanning electron microscope (SEM) image 150 of the dried everolimus particles. Figure 7B is an SEM image 160 of a dried everolimus/polydopamine dispersion that was prepared according to the procedure above and allowed to incubate for one hour. As can be seen, the SEM image 150 of the dried everolimus particles and the SEM image 160 of the dried everolimus/polydopamine particles look about the same. Based on the polydopamine content of the everolimus/polydopamine particles at an incubation time of one hour (approximately 0.5-1%, as shown in Figure 5), the thickness of the polydopamine coating is estimated to be approximately 16 nanometers (nm), assuming an average particle size of approximately 5 µm.

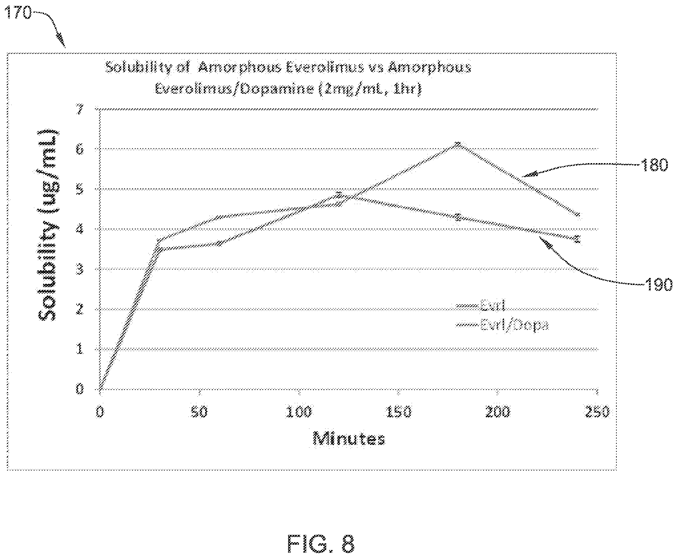

Polydopamine coatings have been shown to be permeable to organic solvents and water (Deposition Mechanism and Properties of Thin Polydopamine Films for High Added Value Applications in Surface Science at the Nanoscale. BioNanoSci. 2012. 2:16-34). The impact of the polydopamine coating on the rate of dissolution of the drug in water was determined. An amorphous everolimus dispersion was treated with 2mg/mL dopamine in THAM buffer at room temperature for 1 hour. Super saturated dispersions of dopamine treated and non-dopamine treated particles were each incubated in water at 37°C for 30 minutes, 1 hour, 2 hours, 3 hours, and 4 hours. After the incubation periods, the dispersions were centrifuged. 1 mL of the supernatant was removed and filtered through a 0.2 µm filter and the everolimus content was determined by HPLC. Figure 8 shows a plot 170 of the dissolution profiles for both uncoated everolimus particles 180 and the polydopamine coated everolimus particles 190. For example, Figure 8 shows the concentration of everolimus in micrograms per milliliter (µg/mL) as a function of incubation time. Both the uncoated 180 and polydopamine coated everolimus particles 190 display similar drug dissolution profiles, as shown in Figure 8. Thus, the nanometer thin polydopamine coating does not inhibit the dissolution of the drug particle.

### Example 2

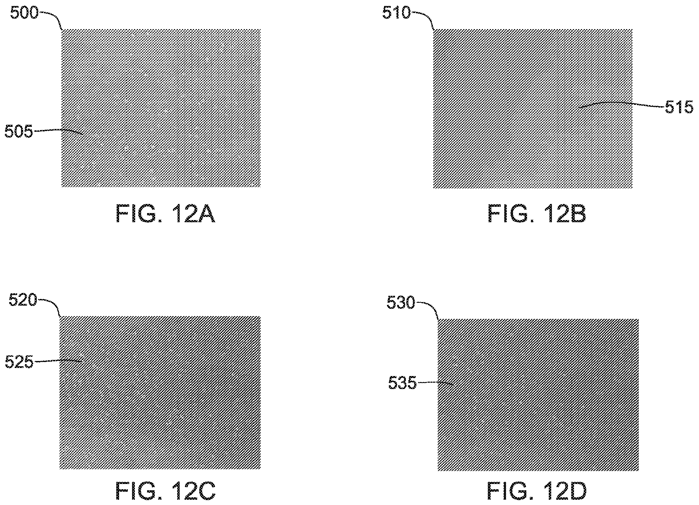

To quantify the impact of polydopamine coating on the adhesion of particles to tissue, polymethylmethacrylate (PMMA) beads (10-20um) containing a fluorescent dye were used as a model for drug microparticles. PMMA beads were prepared by oil/water emulsion. The beads were then treated with 2mg/mL dopamine in tris buffer (pH 8.5) for 1 hour at room temperature. Control beads were also prepared that did not have the polydopamine coating. An approximately 0.25 by 0.25 inch (0.634 centimeter by 0.64 centimeter) square sample of pig aorta was rinsed with deionized water and 1-2 drops of the bead dispersion in water was placed on the artery for 60 sec. The artery was imaged under a microscope with UV black light illumination. Figure 12A is a microscope image 500 of the control beads 505 after being placed on the pig artery. Figure 12C is a microscope image 520 of the polydopamine coated beads 525 after being placed on the pig artery. During this time the beads settled to the surface of the artery. The artery was then rinsed in a beaker of deionized water stirred with a magnetic stir bar at 300 rpm for 30 sec. The artery was then imaged again. Figure 12B is a microscope image 510 of the control beads 515 on the pig after the deionized water rinse. Figure 12D is a microscope image 530 of the polydopamine coated beads 535 on the pig after the deionized water rinse. Image analysis was used to count the beads before and after rinsing to determine the percentage of beads retained on the artery. The results are summarized in Table 1 below:

**Table 1 - Percentage of beads remaining after rinsing**

| **Coating** | **% Beads retained after rinsing** |
|---|---|
| PMMA control | 0.5% |
| PMMA coated with polydopamine | 72% |

As can be seen, in the absence of the polydopamine coating essentially all the microspheres washed off the artery. In contrast, microspheres coated with polydopamine showed an approximately 70% retention of the beads to the artery post rinsing. Thus, the polydopamine coating is able to impart a strong adhesive interaction of the microsphere to the artery within seconds.

### Example 3

Crystalline everolimus microparticles were coated with polydopamine as described in Example 1. A control sample of 2% solids everolimus dispersion in deonized water and a sample of polydopamine coated crystalline everolimus were coated on individual glass slides using a microsyringe to a drug density of about 3ug/mm² and dried for 10 min at 45°C. A flat section of pig aorta was placed on the coated slide and a 45 gram weight was placed on artery for 1 minute to approximate the deployment of a drug coated balloon within an artery. The aorta was then rinsed in a beaker of deionized water stirred with a magnetic stir bar at 300 rpm for 30 sec. The drug content of the artery and glass slide were determined by HPLC The results are summarized in Table 2 below:

**Table 2 - Percentage of drug transfer and retention**

| **Formulation** | **% Drug transferred from glass slide to artery (pre-rinse)** | **% Drug retained on artery (post-rinse)** |
|---|---|---|
| Everolimus control | 70% | 22% |
| Everolimus/polydopamine | 76% | 41% |

Comparing the uncoated drug control (everolimus) and polydopamine coated drug particles, about the same amount of drug is initially transferred to the artery before rinsing. After rinsing, approximately double the amount of drug is retained on the artery in the case of drug particles coated with polydopamine.

### Coating of Balloons

Several 4.0 millimeter (mm) (diameter) by 16 mm (length) Liberté™ component balloons (manufactured by Boston Scientific Corporation, Marlborough, Massachusetts) were syringe coated by hand with either an everolimus dispersion or an everolimus-polydopamine dispersion. One set of balloons was coated with the dispersions (everolimus or everolimus/polydopamine) to a dose of 3 µg drug/mm². In another set the balloons were 1st coated with acetyltributylcitrate (ATBC) to a dose of 0.75 µg/mm². As one of ordinary skill in the art may be aware, ATBC may help release the drug from the balloon. The balloons were then overcoated with the everolimus or everolimus-polydopamine dispersions to a dose of 3 µg /mm². The coated balloons were immersed in water for one minute and the coating was scraped off of the balloon with the edge of a spatula into the water. A control balloon, from the Agent™ Paclitaxel-coated PTCA balloon catheter (manufactured by Boston Scientific Corporation, Marlborough, Massachusetts) was also immersed in water for one minute. The coating was then scraped off of the balloon with the edge of a spatula into the water. The vials with the coatings were imaged to qualitatively show the resulting particulates.

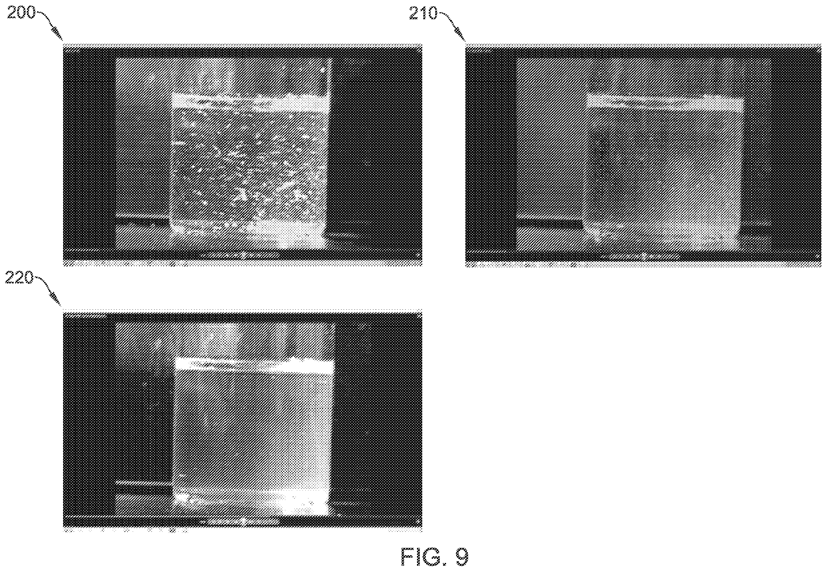

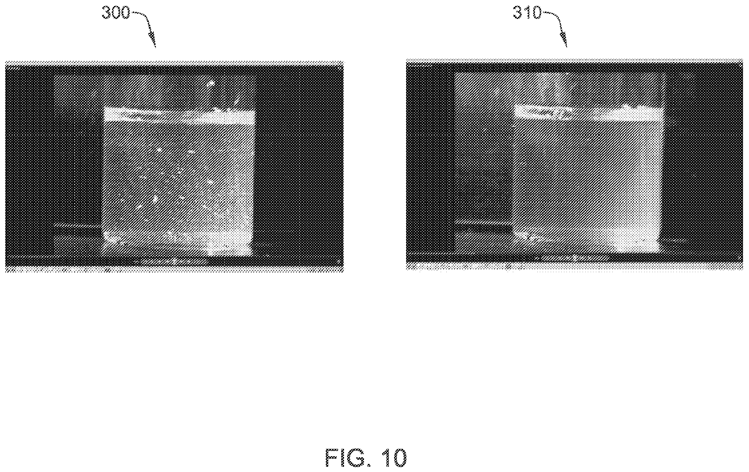

Figure 9 shows the coating particulates in water for the Agent™ Paclitaxel-coated PTCA balloon 200, the everolimus over ATBC control balloon 210, and the everolimus-polydopamine over ATBC balloon 220. As can be seen, both the everolimus over ATBC control balloon 210 and the everolimus-polydopamine over ATBC balloon 220 show significantly smaller particulates than the Agent™ Paclitaxel-coated PTCA balloon 200, Figure 10 shows the coating particulates in water for the everolimus control balloon 300 and the everolimus-polydopamine balloon 310. It appears that the particles of everolimus-polydopamine 220, 310 are smaller than the everolimus controls 210, 300 (which show larger particle agglomerates). This is likely due to the polydopamine coating preventing the everolimus particles from agglomerating in the dried coating and upon subsequent rehydration. It may be desirable for the drug particulates to be small in size as large particulates may create embolisms.

The materials that can be used for the various components of catheter 10 (and/or other devices disclosed herein) and the various tubular members disclosed herein may include those commonly associated with medical devices. For simplicity purposes, the following discussion makes reference to catheter 10. However, this is not intended to limit the devices and methods described herein, as the discussion may be applied to other similar medical devices and/or components of medical devices disclosed herein.

Catheter 10 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKEL VAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803, which are incorporated herein by reference. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of catheter 10 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of catheter 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of catheter 10 to achieve the same result.

In some embodiments, a degree of Magnetic Resonance Imaging (MRI) compatibility is imparted into catheter 10. For example, catheter 10, or portions thereof, may be made of a material that does not substantially distort the image and create substantial artifacts (i.e., gaps in the image). Certain ferromagnetic materials, for example, may not be suitable because they may create artifacts in an MRI image. Catheter 10, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

It should be understood that this disclosure is, in many respects, only illustrative. Changes may be made in details, particularly in matters of shape, size, and arrangement of steps without exceeding the scope of the disclosure. The invention's scope is, of course, defined in the language in which the appended claims are expressed.

## Claims

1. An expandable medical device comprising:
a therapeutic coating comprising a polydopamine coated therapeutic agent disposed on a surface of the expandable medical device.

2. The expandable medical device of claim 1, wherein the therapeutic agent is selected from the group consisting of paclitaxel, everolimus, rapamycin, sirolimus, tacrolimus, heparin, diclofenac, aspirin, and any combination thereof.

3. The expandable medical device of any one of claims 1 or 2, wherein the therapeutic agent has an average particle size in the range of 0.1 - 20 micrometers.

4. The expandable medical device of any one of claims 1-3, wherein the polydopamine has a thickness in the range of 1 - 40 nanometers.

5. The expandable medical device of any one of claims 1-4, wherein the expandable medical device is a balloon catheter

6. The expandable medical device of claim 5, wherein the balloon catheter comprises:
a catheter shaft comprising an inner tubular member and an outer tubular member, the catheter shaft extending from a proximal end to a distal end; and
an inflatable balloon having a proximal end affixed to the outer tubular member of the catheter shaft and a distal end affixed to the inner tubular member of the catheter shaft proximal to the distal end of the catheter shaft, the inflatable balloon disposed adjacent to the distal end of the catheter shaft.

7. The expandable medical device of claim 6, wherein the therapeutic coating is disposed over an entire outer surface area of the inflatable balloon.

8. The expandable medical device of claim 6, wherein the therapeutic coating is disposed over less than an entire outer surface area of the inflatable balloon.

9. The expandable medical device of any one of claims 1-4, wherein the expandable medical device is a stent.

10. The expandable medical device of claim 9, wherein the stent comprises:
an elongated tubular member having a first end, a second end and an intermediate region disposed therebetween, the elongated tubular member comprising at least one filament.

11. The expandable medical device of any one of claims 9-10, wherein the therapeutic coating is disposed on an outer surface of the stent.

12. The expandable medical device of any one of claims 9-11, wherein the therapeutic coating is disposed on an inner surface of the stent.

13. The expandable medical device of any one of claims 9-12, wherein the therapeutic coating is disposed over an entire surface area of the stent.

14. The expandable medical device of any one of claims 9-12, wherein the therapeutic coating is disposed over less than an entire surface area of the stent

15. The expandable medical device of any one of claims 1-14, wherein the therapeutic coating binds with primary amines.

## Patentansprüche

1. Expandierbare medizinische Vorrichtung, umfassend:
eine therapeutische Beschichtung, umfassend ein mit Polydopamin beschichtetes therapeutisches Mittel, das auf einer Oberfläche der expandierbaren medizinischen Vorrichtung angeordnet ist.

2. Expandierbare medizinische Vorrichtung nach Anspruch 1, wobei das therapeutische Mittel ausgewählt ist aus der Gruppe bestehend aus Paclitaxel, Everolimus, Rapamycin, Sirolimus, Tacrolimus, Heparin, Diclofenac, Aspirin und jeglicher Kombination davon.

3. Expandierbare medizinische Vorrichtung nach einem der Ansprüche 1 oder 2, wobei das therapeutische Mittel eine durchschnittliche Partikelgröße im Bereich von 0,1 bis 20 Mikrometern aufweist.

4. Expandierbare medizinische Vorrichtung nach einem der Ansprüche 1 bis 3, wobei das Polydopamin eine Dicke im Bereich von 1 bis 40 Mikrometern aufweist.

5. Expandierbare medizinische Vorrichtung nach einem der Ansprüche 1 bis 4, wobei die expandierbare medizinische Vorrichtung ein Ballonkatheter ist.

6. Expandierbare medizinische Vorrichtung nach Anspruch 5, wobei der Ballonkatheter umfasst:
einen Katheterschaft, der ein inneres rohrförmiges Element und ein äußeres rohrförmiges Element umfasst, wobei der Katheterschaft sich von einem proximalen Ende zu einem distalen Ende erstreckt; und
einen befüllbaren Ballon mit einem proximalen Ende, das an dem äußeren rohrförmigen Element des Katheterschafts angebracht ist, und einem distalen Ende, das an dem inneren rohrförmigen Element des Katheterschafts proximal zu dem distalen Ende des Katheterschafts angebracht ist, wobei der befüllbare Ballon benachbart zu dem distalen Ende des Katheterschafts angeordnet ist.

7. Expandierbare medizinische Vorrichtung nach Anspruch 6, wobei die therapeutische Beschichtung über einem gesamten äußeren Oberflächenbereich des befüllbaren Ballons angeordnet ist.

8. Expandierbare medizinische Vorrichtung nach Anspruch 6, wobei die therapeutische Beschichtung über weniger als einem gesamten äußeren Oberflächenbereich des befüllbaren Ballons angeordnet ist.

9. Expandierbare medizinische Vorrichtung nach einem der Ansprüche 1 bis 4, wobei die expandierbare medizinische Vorrichtung ein Stent ist.

10. Expandierbare medizinische Vorrichtung nach Anspruch 9, wobei der Stent umfasst:
ein längliches rohrförmiges Element mit einem ersten Ende, einem zweiten Ende und einer Zwischenregion, welche dazwischen angeordnet ist, wobei das längliche rohrförmige Element mindestens ein Filament umfasst.

11. Expandierbare medizinische Vorrichtung nach einem der Ansprüche 9 bis 10, wobei die therapeutische Beschichtung auf einer äußeren Oberfläche des Stents angeordnet ist.

12. Expandierbare medizinische Vorrichtung nach einem der Ansprüche 9 bis 11, wobei die therapeutische Beschichtung auf einer inneren Oberfläche des Stents angeordnet ist.

13. Expandierbare medizinische Vorrichtung nach einem der Ansprüche 9 bis 12, wobei die therapeutische Beschichtung über einem gesamten Oberflächenbereich des Stents angeordnet ist.

14. Expandierbare medizinische Vorrichtung nach einem der Ansprüche 9 bis 12, wobei die therapeutische Beschichtung über weniger als einem gesamten Oberflächenbereich des Stents angeordnet ist.

15. Expandierbare medizinische Vorrichtung nach einem der Ansprüche 1 bis 14, wobei die therapeutische Beschichtung mit primären Aminen bindet.

## Revendications

1. Dispositif médical expansible comprenant :
un revêtement thérapeutique comprenant un agent thérapeutique revêtu de polydopamine disposé sur une surface du dispositif médical expansible.

2. Dispositif médical expansible selon la revendication 1, l'agent thérapeutique étant choisi dans le groupe constitué par le paclitaxel, l'évérolimus, la rapamycine, le sirolimus, le tacrolimus, l'héparine, le diclofénac, l'aspirine et toute combinaison de ceux-ci.

3. Dispositif médical expansible selon l'une quelconque des revendications 1 et 2, l'agent thérapeutique ayant une taille moyenne de particules dans la gamme de 0,1 à 20 micromètres.

4. Dispositif médical expansible selon l'une quelconque des revendications 1 à 3, la polydopamine ayant une épaisseur dans la gamme de 1 à 40 nanomètres.

5. Dispositif médical expansible selon l'une quelconque des revendications 1 à 4, le dispositif médical expansible étant un cathéter à ballonnet.

6. Dispositif médical expansible selon la revendication 5, le cathéter à ballonnet comprenant :
une tige de cathéter comprenant un élément tubulaire intérieur et un élément tubulaire extérieur, la tige de cathéter s'étendant d'une extrémité proximale à une extrémité distale ; et
un ballonnet gonflable ayant une extrémité proximale fixée à l'élément tubulaire extérieur de la tige de cathéter et une extrémité distale fixée à l'élément tubulaire intérieur de la tige de cathéter à proximité de l'extrémité distale de la tige de cathéter, le ballonnet gonflable étant disposé de manière adjacente à l'extrémité distale de la tige de cathéter.

7. Dispositif médical expansible selon la revendication 6, le revêtement thérapeutique étant disposé sur la totalité d'une surface extérieure du ballonnet gonflable.

8. Dispositif médical expansible selon la revendication 6, le revêtement thérapeutique étant disposé sur moins de la totalité d'une surface extérieure du ballonnet gonflable.

9. Dispositif médical expansible selon l'une quelconque des revendications 1 à 4, le dispositif médical expansible étant un stent.

10. Dispositif médical expansible selon la revendication 9, le stent comprenant :
un élément tubulaire allongé ayant une première extrémité, une seconde extrémité et une région intermédiaire disposée entre elles, l'élément tubulaire allongé comprenant au moins un filament.

11. Dispositif médical expansible selon l'une quelconque des revendications 9 et 10, le revêtement thérapeutique étant disposé sur une surface extérieure du stent.

12. Dispositif médical expansible selon l'une quelconque des revendications 9 à 11, le revêtement thérapeutique étant disposé sur une surface intérieure du stent.

13. Dispositif médical expansible selon l'une quelconque des revendications 9 à 12, le revêtement thérapeutique étant disposé sur la totalité d'une surface du stent.

14. Dispositif médical expansible selon l'une quelconque des revendications 9 à 12, le revêtement thérapeutique étant disposé sur moins de la totalité d'une surface du stent.

15. Dispositif médical expansible selon l'une quelconque des revendications 1 à 14, le revêtement thérapeutique se liant à des amines primaires.
